# EUROPÄISCHE PATENTSCHRIFT

(11) **EP 2 042 199 B1**
(45) Veröffentlichungstag und Bekanntmachung des Hinweises auf die Patenterteilung: **17.04.2013**
(21) Anmeldenummer: 07023973.6
(22) Anmeldetag: 11.12.2007
(51) Int. Cl.: D01F 1/10, D01F 4/00, D01F 9/00, D04H 1/70, D04H 1/02, D04H 3/03, D04H 1/736, D04H 1/728, D04H 3/016, A61L 15/46, A61L 15/32, D04H 3/00, D01D 5/18, D04H 1/42

(54) **Bioresorbierbare Wundauflagen**
Bioresorbable wound dressings
Pansements biorésorbables

(30) Priorität: 18.09.2007 DE 102007044648
(43) Veröffentlichungstag der Anmeldung: 01.04.2009
(73) Patentinhaber: Carl Freudenberg KG, 69469 Weinheim (DE)
(72) Erfinder: Reibel, Denis, 67850 Herrlisheim (FR); Schlesselmann, Bernd, 69469 Weinheim (DE); Kerhault, Jean-Francois, 68766 Hockenheim (DE); Groten, Robert, 68280 Sundhoffen (FR)

(56) Entgegenhaltungen:
- WO-A1-2004/002384
- WO-A1-2005/054553
- WO-A2-2007/110783
- WO-A2-2007/122232
- DE-A1-102005 048 939
- US-A1- 2004 110 439

## Beschreibung

### Technisches Gebiet

Die Erfindung betrifft ein Rotationsspinnverfahren zur Herstellung Vliesstoffs umfassend Fasern aus einem Faserrohmaterial, wobei die Fasern mit einer biologisch aktiven Substanz ausgerüstet sind und die biologisch aktive Substanz in den Fasern verteilt ist und dessen Verwendung.

### Stand der Technik

Vliesstoffe, die mit biologisch aktiven Substanzen ausgerüstet sind, sind bereits aus dem Stand der Technik bekannt. Insbesondere zeigt die WO 2004/002384 A1 einen Vliesstoffe, welcher Silber aufweist und als Wundauflage verwendet wird. Als Faser Material werden dort Polyurethane oder Polyacrylate vorgeschlagen.

Vliesstoffe werden häufig für medizinische Anwendungen verwendet. Den als, Rohmaterial für die Vliesstoffe vorliegende Vliesen wird durch Wasserstrahlverfestigung, thermische oder chemische Verfestigung eine hinreichende Reißfestigkeit verliehen, damit diese als Verbandsmaterial eingesetzt werden können.

Die mechanischen oder chemischen Verfestigungsverfahren können jedoch die Ausrüstung der Fasern mit biologischen Wirkstoffen, beispielsweise antimikrobiellen oder antibiotischen Wirkstoffen, negativ beeinträchtigen, nämlich die wirksamen Stoffe in ihrer Wirkung hemmen oder sogar teilweise aus den Fasern herauslösen. Daher sind häufig aufwendige und teure Nachbehandlungsschritte notwendig, um die verfestigten Vliesstoffe in einen gebrauchstauglichen Zustand zu verbringen.

Des Weiteren ist nachteilig, dass Faserrohmaterial aus Polymeren, die eine ausreichende Verfestigung erlauben, häufig nicht wundverträglich, insbesondere nicht bioresorbierbar ist.

Für die Aufbringung biologisch aktiver Substanzen auf Vliesstoffe werden häufig Verfahren verwendet, bei denen der Vliesstoff nach seiner Herstellung mit den Substanzen behandelt wird. Dies hat den Nachteil, das mehrstufige Verfahren erforderlich sind, bei denen die Herstellung des Vliesstoffes gesondert von der Aufbringung der Substanzen erfolgt. Zudem wird bei solchen Verfahren regelmäßig nicht eine gleichmäßige, stabile Verteilung der Substanzen in dem Vliesstoff erzielt. Daher müssen oft die Ärzte oder das medizinische Personal die Aufbringung der aktiven Substanzen auf die industriell hergestellten Vliesstoffe vornehmen.

Ein weiteres Problem ist es, das im Falle einer mechanischen Einarbeitung von biologisch aktiven Substanzen in die Vliesstoffe, insbesondere wenn die Substanzen zusammen mit einem Faserrohmaterial zu einem Vliesstoff verarbeitet werden, sowohl die Fasern als auch die biologisch aktiven Substanzen geschädigt werden können. Die Schädigung erfolgt vor allem durch die Hitzebehandlung bei der Herstellung der Vliesfasern. Daher sind solche Verfahren bislang für die Herstellung von Vliesstoffen aus therminostabilen Substanzen wie Polysacchariden und Polypeptiden und für die Einarbeitung von thermolabilen biologischen Substanzen wie Enzymen oder Zellen nicht geeignet.

Der Erfindung liegt die Aufgabe zugrunde, Vliesstoffe und Verfahren zu ihrer Herstellung zur Verfügung zu stellen, die die beschriebenen Nachteile überwinden.

Der Erfindung liegt insbesondere die Aufgabe zugrunde, ein einfaches Verfahren zur Herstellung von Vliesstoffen zur Verfügung zu stellen, die mit biologischen Wirkstoffen ausgerüstet sind. Insbesondere soll das Verfahren die Herstellung von Vliesstoffen und deren Ausrüstung mit biologisch aktiven Wirkstoffen ermöglichen, die wegen ihrer Instabilität, besonderes gegenüber Hitze, nach bekannten Verfahren nicht herstellbar sind.

Der Erfindung liegt die weitere Aufgabe zugrunde, einen bioresorbierbaren Vliesstoff mit hinreichender Festigkeit kostengünstig zu fertigen.

### Darstellung der Erfindung

Die vorliegende Erfindung löst die zuvor genannte Aufgabe durch die Merkmale der Patentansprüche 1 bis 25.

Überraschenderweise erlaubt das erfindungsgemäße Verfahren die gleichmäßige Einarbeitung biologisch aktiver Substanzen in Vliesstoffe während der Erzeugung der Vliese in einem Rotationsspinnverfahren. Da bei dem Rotationsspinnverfahren die Faserrohmaterialien und die biologisch aktiven Substanzen sehr kurze Verweilzeiten in der Spinvorrichtung aufweisen, die beispielsweise im Bereich von Mikrosekunden liegen können, werden weder die Faserrohmeterialien noch die aktiven Substanzen geschädigt. Auf diese Art werden neue Vliesstoffe erhalten, die nach bekannten Verfahren nicht herstellbar waren. Bei den bekannten Elektrospinnverfahren sind Verweildauern von mehreren Minuten üblich.

Erfindungsgemäß ist insbesondere erkannt worden, dass beispielsweise Gelatine und Dextrane biologisch abbaubare Materialien sind, die sich überraschend gut zu einem Vlies ablegen lassen, wobei sich gleichzeitig in die Fasern biologische Wirkstoffe einarbeiten lassen. Des Weiteren wurde erkannt, dass die Fasern des Vlieses zum Teil ohne Phasengrenze ineinander übergehen, miteinander vernetzen und dadurch einen festen Verbund ausbilden. Der entstehende Vliesstoff zeigt ohne weitere Verfestigungsmaßnahmen überraschend eine hinreichend hohe Reißfestigkeit, um als Verbandsmaterial oder Wundauflage Verwendung zu finden. Die biologisch aktive Substanz wird in ihrer Wirkung nicht durch Verfestigungsmaßnahmen negativ beeinträchtigt. Weiter ist erkannt worden, dass mittels eines Rotationsspinnverfahrens der Durchmesser der Fasern in einer engen Verteilung einstellbar ist. Durch das Rotationsspinnverfahren sind Fasern mit einem Durchmesser von im Mittel 0,3 bis 500 µm, im Mittel 3 bis 200 µm und sogar im Mittel 5 bis 100 µm erzeugbar. Die enge Verteilung des Durchmessers der Fasern sowie die Steuerung der Restfeuchte erlauben einen homogenen und stabilen Aufbau des Vliesstoffs ohne teure zusätzliche Verfestigungsmaßnahmen.

Folglich ist die eingangs genannte Aufgabe gelöst.

Gegenstand der Erfindung ist insbesondere Rotationsspinnverfahren zur Herstellung eines Vliesstoffs, umfassend Fasern aus einem Faserrohmaterial, das natürliche Polymere aufweist, wobei die Fasern mit einer biologisch aktiven Substanz ausgerüstet sind und die biologisch aktive Substanz in den Fasern verteilt ist. Im Sinne der Erfindung bedeutet biologisch aktive Substanz", dass die Substanz gezielt chemisch auf biologische Prozesse einwirkt, wie beispielsweise das Wachstum von Bakterien oder Keimen oder die Wundheilung.

Ein weiterer Gegenstand der Erfindung ist ein Vliesstoff, umfassend Fasern aus einem Faserrohmaterial, wobei die Fasern mit einer biologisch aktiven Substanz ausgerüstet sind, wobei die Fasern durch das Rotationsspinnverfahren gemäss Anspruch 1 hergestellt sind.

In einer bevorzugten Ausführungsform der Erfindung sind das Faserrohmaterial und/oder die biologisch aktive Substanz oberhalb 150 °C oder oberhalb 100 °C instabil, "Instabil" bedeutet, dass das Material sich innerhalb von 1 Minute, insbesondere 10 Sekunden, bei dieser Temperatur so verändert, dass die Verwendbarkeit als Fasermaterial zur Erzeugung eines Vliesstoffes oder die biologische Aktivität signifikant abnimmt. "Signifikant" bedeutet dabei beispielsweise, dass die biologische Aktivität um mindestens 10% abnimmt. Beispielsweise neigen Enzyme bei solchen Temperaturen zum Denaturieren, während sich instabile Antibiotika chemisch zersetzen. Fasermaterialien wie Polysaccharide, Proteine, Polypeptide oder Gelatine neigen bei erhöhten Temperaturen zum Denaturieren oder Verklumpen.

Erfindungsgemäß sind die Faserrohmaterialien bevorzugt ausgewählt aus der Gruppe bestehend aus natürlichen Polymeren, synthetischen Polymeren und Polymeren aus fossilen Rohmaterialien, jeweils in unmodifizierter und/oder modifizierter Form.

"Natürliche Polymere" im Sinne der Erfindung sind solche, die ursprünglich aus biologischen Quellen wie pflanzlichem, tierischem oder bakteriellem Material stammen, wobei die Bezeichnung nachbehandelte und chemisch modifizierte Polymere einschließt. In bevorzugten Ausführungsformen der Erfindung sind die natürlichen Polymere Polypeptide, Polysaccharide, Polyhydroxyester und Polynucleotide.

Bevorzugt einsetzbare Polypeptide sind Gelatine, Collagen, Fibrin oder Casein. Besonders bevorzugt ist Gelatine, die ein Gemisch von Polypeptiden ist, die üblicherweise aus tierischem Bindegewebe hergestellt wird.

In bevorzugten Ausführungsformen der Erfindung ist das Polysaccharid Dextran, Chitosan, Chitin, Cellulose, Stärke, ein Hyaluronsäurederivat oder ein Alginat. Besonders bevorzugt sind Dextrane. Dabei handelt es sich um hochmolekulare, neutrale Biopolysaccharide auf der Basis von Glucose-Monomeren. Sie werden beispielsweise von Bakterien mittels Enzymen extrazellulär aus Saccharose hergestellt.

Der erfindungsgemäße Vliesstoff ist besonders bevorzugt bioresorbierbar. Dies bedeutet, dass der Vliesstoff im oder am Körper abbaubar ist. Solchen Materialien müssen vorzugsweise nach der Anwendung bei vollständiger Resoption nicht mehr entfernt werden und sind oft besonders gut vom Organismus verträglich.

In einer weiteren bevorzugten Ausführungsform ist der Vliesstoff biologisch abbaubar. Dies bedeutet, dass der Vliesstoff von natürlichen Organismen, beispielsweise Bakterien oder Pilzen, zersetzt werden kann. Ein solcher Abbau erfolgt üblicherweise innerhalb von Tagen, Wochen oder Monaten. Solche Materialien sind bespielsweise aus Umweltschutzgründen vorteilhaft.

Biologisch abbaubare oder bioresorbierbare Polymere sind beispielsweise Alginate aus Algen, natürliche Polysaccharide wie Dextran, die pflanzlichen Polymere Stärke und Cellulose, die tierischen Polymere Collagen, Gelatine, Chitin, Chitosan, Casein, Polydepsipepfide, bakterielle Polymere wie Polyhydroxyester, insbesondere Polyhydroxybutyrate und -valerate, synthetische Polymere auf der Basis von Pflanzenöl wie Polymilchsäure, Polyglycolsäure, Polyamide und Polyurethane, sowie Polymere aus fossilen Rohmaterialien wie Poly-ε-Caprolacton, Polyvinylalkohol, Polyester, Polyethylensuccinat und -oxalat, Polyesteramide, Nylon 2/Nylon 6 Copolyamide und Polydioxanon.

Bei den erfindungsgemäßen Vliesstoffen handelt es sich um nicht gewebte ("non woven") Materialien. Diese sind bevorzugt nicht nachverdichtet.

Einige Fasern, bevorzugt mehr als 5 oder 20% der Fasern, könnten miteinander verdrillt sein oder eine verdrillte Struktur aufweisen, Die Verdrillungen stellen sich überraschend während des Rotationsspinnens ein und begünstigen zusätzlich die Festigkeit des Vliesstoffs.

Die Fasern sind in einer besonders bevorzugten Ausführungsform der Erfindung ausschließlich aus Gelatine, Dextrane oder Derivaten der Gelatine gefertigt, wobei in und/oder auf den Fasern ein antimikrobieller Stoff und/ oder ein Antibiotikum vorliegt. Ein solcher Vliesstoff kann durch die Chemie des menschlichen Körpers abgebaut werden und ist daher nahezu vollständig bioresorbierbar. Dieser Vliesstoff kann in den menschlichen Körper eingebracht werden.

Die biologisch aktive Substanz ist in den Fasern verteilt. Hierdurch lässt sich eine allmähliche Abgabe der biologisch aktiven Substanz mit lang anhaltender Wirkung einstellen. Dabei schließt die Verteilung "in den Fasern" nicht aus, dass die Substanz zusätzlich auf den Fasern, also an deren Oberfläche gebunden, vorliegt. Bevorzugt ist die Verteilung in den Fasern homogen. "Homogen" bedeutet dabei, dass die aktive Substanz im Gegensatz zu Vliesen, die mit Wirkstoffen nachbehandelt werden, innerhalb der Fasern im wesentlichen gleichmäßig eingeschlossen ist und nicht ungleichmäßig verteilt ist, beispielsweise aufgrund einer unvollständigen Diffusion in die Fasern. Dem steht nicht entgegen, dass sich möglicherweise erhöhte Mengen der Wirkstoffe an der Faseroberfläche befinden, beispielsweise wegen Grenzflächeneffekten.

Die biologisch aktive Substanz könnte in den Fasern nanoskalig vorliegen. Unter nanoskaligen Strukturen versteht man Bereiche jeglicher Morphologie, die zumindest in einer Raumrichtung Dimensionen im Nanometerbereich aufweisen. Hierdurch erlangt die biologisch aktive Substanz eine hohe Mobilität. Eine nanoskalig vorliegende biologisch aktive Substanz zeigt eine besonders hohe Reaktivität, wenn sie mit Bakterien, Viren, Pilzen oder Sporen in Kontakt gebracht wird. Des Weiteren gibt der Vliesstoff den wirkenden Stoff sehr leicht an Medien ab, die mit diesem in Kontakt treten. Insoweit zeichnet ' sich der Vliesstoff durch eine hohe Abgabefähigkeit in Bezug auf die biologisch aktive Substanz aus.

In einer bevorzugten Ausführungsform der Erfindung ist die biologisch aktive Substanz zusätzlich auf den Fasern verteilt, um eine rasche Abgabe an den menschlichen Körper sicher zu stellen. Dies wird durch ein Besprühen des Vliesstoffs mit der Substanz oder ein Tränken in der Substanz ermöglicht.

In bevorzugten Ausführungsformen der Erfindung ist die biologisch aktive Substanz ein Antiseptikum, ein antimikrobiell wirksamer Stoff, ein Antibiotikum, ein Medikament, ein Wachstumsfaktor, ein Enzym und/oder eine Zelle. Als "antiseptisch" bezeichnet man eine Wirkung, die zur Verminderung der Anzahl von infektiösen Keimen und damit zur Verhinderung einer Infektion führt. Als "Substanz'" im Sinne der Verbindungen werden nicht nur einzelne chemische Verbindungen verstanden, sondern auch Substanzgemische oder komplexe Einheiten wie Zellen oder Extrakte.

In einer bevorzugten Ausführung der Erfindung wird die biologisch aktive Substanz in Kombination mit geeigneten Trägerstoffen, Stabilisatoren oder sonstigen galenischen Zusätzen in das Vlies eingearbeitet.

Der antimikrobiell wirksame Stoff könnte Silber enthalten. Silber ist besonders als antimikrobiell wirkender Stoff geeignet, da es für Menschen nahezu ungiftig ist. Des Weiteren zeigt Silber ein relativ geringes allergenes Potential, Silber wirkt als antiseptische Substanz in geringen Konzentrationen über einen langen Zeitraum auf eine Vielzahl von Infektionskeimen. Die meisten bekannten Bakterien zeigen darüber hinaus keine Resistenz gegen Silber.

Der Stoff könnte zumindest ein Nebengruppenelement umfassen. Nebengruppenelemente zeichnen sich durch antimikrobielle Wirkung aus. Vor diesem Hintergrund ist denkbar, dass mehrere Nebengruppenelemente gemeinsam vorliegen, um unterschiedlichen Bakterienarten selektiv zu begegnen. Es hat sich in Versuchsreihen gezeigt, dass sich in Bezug auf die antimikrobielle Wirksamkeit eine Rangfolge der verwendeten Stoffe ergibt. Diese lässt sich wie folgt darstellen. Silber ist der wirksamste Stoff, gefolgt von Quecksilber, Kupfer, Cadmium, Chrom, Blei, Kobalt, Gold, Zink, Eisen und schließlich Mangan. Vor diesem Hintergrund ist denkbar, auch Hauptgruppenelemente zu verwenden, welche eine antimikrobielle Wirkung zeigen. Der antimikrobieil wirkende Stoff könnte eine Gold-Silber Mischung umfassen oder ausschließlich aus einer Gold-silber-Mischung bestehen. Mischungen dieser Art zeigen eine besonders hohe antimikrobielle Wirksamkeit. Es hat sich überraschenderweise gezeigt, dass die Anwesenheit von Gold die antimikrobielle Wirkung von Silber noch steigert,

Zumindest ein Teil der Fasern könnte als Nanofasem ausgestaltet sein. Ein Vliesstoff dieser Ausgestaltung kann besonders leicht und dünn ausgestaltet sein.

Der Vliesstoff könnte eine Reißfestigkeit bei einem spezifischen Flächengewicht von 140 bis 180 g/m² im trockenen Zustand von 0,1 N/mm² oder mehr und eine Reißdehnung in hydratisiertem Zustand von 100%, bevorzugt von 200% oder mehr aufweisen. Ein solcher Vliesstoff eignet sich besonders gut als Verbandsmaterial, da er problemlos aufgewickelt werden kann.

Der Vliesstoff könnte eine offene Porenstruktur mit einer Luftdurchlässigkeit von 0,5 l/min*cm² aufweisen, wobei dieser Parameter gemäß DIN 9237 ermittelt wird. Ein solcher Vliesstoff eignet sich besonders gut als Verbandsmaterial, da er der Haut erlaubt, Feuchtigkeit abzugeben und zu atmen.

Die eingangs genannte Aufgabe wird auch durch die Merkmale des erfindungsgemäßen Verfahrens gelöst. Um Wiederholungen in Bezug auf die erfinderische Tätigkeit zu vermeiden, sei auf die Ausführungen zum Vliesstoff als solchem verwiesen.

Die austretenden Fasern könnten gerichtet, kontaktlos geführt werden. Eine kontaktlose und definierte Führung der Fasern, bevor diese auf einer Ablageeinrichtung auftreffen, erlaubt eine Modifikation der Fasern. So kann allein die Dauer der Führung bzw. die Richtung der Führung Einfluss auf die Faserlänge, den Faserdurchmesser sowie die Faserstruktur nehmen. Eine gerichtete kontaktlose Führung erzeugt ein homogeneres Faserspektrum, als ein Nerstellungsprozess ohne Führung. Ganz konkret kann allein durch die Führung die Breite einer Verteilungskurve sämtlicher Fasereigenschaften eingestellt werden. Hierdurch kann die Menge an Fasern mit unerwünschter Fasergeometrie erheblich reduziert werden.

In einer konstruktiv besonders günstigen Ausgestaltung könnten die Fasern durch eine Absaugeinrichtung geführt werden. Dabei ist denkbar, dass die Fasern durch einen Gasstrom transportiert werden. Sofern der Gasstrom laminar ausgebildet ist, können die Fasern zwischen den laminaren Schichten gestreckt und geformt werden.

Als Gas könnte Luft fungieren. Luft ist ein kostengünstiges Gas, welches sich dadurch auszeichnet, dass ein Herstellungsprozess bei Atmosphärendruck durchgeführt werden könnte. Denkbar ist auch, dass anstelle von Luft weitere Gase, insbesondere Inertgase wie Stickstoff verwendet werden. Hierdurch ist sicher gestellt, dass das Faserrohmaterial verarbeitbar ist, welches reaktive Gruppen umfasst oder zu Nachreaktionen nach dem Verlassen des Behältnisses neigt.

Es könnten Fasern hergestellt werden, die einen Durchmesser von 0,3 bis 500 µm aufweisen. Insoweit ist eine Herstellung von Nanofasem möglich, ohne dass ein elektrostatisches Feld verwendet werden muss.

Für die Durchführung des Rotationsspinnvertahrens wird besonders bevorzugt eine Vorrichtung oder ein Behältnis verwendet, wie es in der DE 102005048939 A1 beschrieben wird. Auf diese Vorrichtungen und Behältnisse wird hier ausdrücklich Bezug genommen. Eine Vorrichtung gemäß DE 102005048939 A1 umfasst ein Behältnis zur Aufnahme von Faserrohmaterial, wobei das Behältnis durch mindestens eine Wärmequelle erwärmbar ist, wobei das Behältnis in Rotation versetzbar ist und wobei das Behältnis Austrittsbereiche für das Faserrohmaterial aufweist, dadurch gekennzeichnet, dass den Austrittsbereichen Mittel zur gerichteten, kontaktlosen Führung des aus dem Behältnis austretenden Faserrahmaterials zugeordnet sind.

Die Austrittsbereiche des Behältnisses könnten als Durchgänge ausgestaltet sein. Hierbei ist denkbar, dass die Durchgänge kreisförmig, oval oder rechteckförmig ausgestaltet sind. Ganz in Abhängigkeit von der Form der Durchgänge kann auf die Fasergeometrie Einfluss genommen werden.

Die Durchgänge könnten einen Durchmesser oder eine Weite bis 500 µm aufweisen. Diese Dimensionierung hat sich als besonders vorteilhaft für die Erzeugung von Nanofasem und Mikrofasern erwiesen. Die Durchgänge könnten in einem Abstand von einem Zentimeter voneinander positioniert sein. Denkbar ist auch, dass die Durchgänge in mehreren Reihen übereinander angeordnet sind. Hierdurch ist der Durchsatz an Faserrohmaterial auf besonders einfache Weise steigerbar. Durch Verringerung oder Vergrößerung des Durchmessers ist es möglich, Nano- bzw. Mikrofasern im Bereich von 0,3 bis 500 µm zu erzeugen.

Das Behältnis könnte mit bis zu 25.000 Umdrehungen pro Minute rotierbar sein. Durch diese hohe Drehzahl ist es möglich, Nanofasem mit einem Durchmesser von höchstens 100 nm zu erzeugen. Üblicherweise werden Nanofasem durch ein elektrisches Spinnverfahren unter Verwendung eines elektrischen Feldes erzeugt. Durch eine geeignete konstruktive Ausgestaltung des Behältnisses sowie sehr hohe Umdrehungszahlen ist es jedoch möglich, ohne elektrisches Feld Nanofasem zu erzeugen. Durch Wahl der Rotationsgeschwindigkeit und Viskosität des Faserrohmaterials können auch Fasern mit größerem Durchmesser erzeugt werden.

In der Erfindung ist die durchschnittliche Verweilzeit des Faserrohmaterials in dem Behältnis so kurz, dass die Faserrohmaterialien und/oder die biologisch aktiven Substanzen nicht in unerwünschter Weise verändert wird. Daher nimmt die Verwendbarkeit des Fasermaterials oder die biologische Aktivität der Substanz auch bei einer hohen Temperatur nicht signifikant ab. Die durchschnittliche Verweilzeit ist kürzer als 10 Sekunden, insbesondere kleiner als 1 Sekunde. In besonders bevorzugten Ausführungsformen der Erfindung ist die Verweilzeit kürzer als 100, 10 oder 2 Mikrosekunden. Bei derartig kurzen Verweilzeiten können überraschenderweise auch biologisch aktive Substanzen wie Proteine, hitze-instabile Antibiotika, Wachstumsfaktoren oder Zellen in Fasern eingearbeitet werden, insbesondere auch in hitzeempfindliche Faserrohmaterialien. In einer besonders bevorzugten Ausführungsform werden Hitze-instabile Substanzen in Hitze-instabile Fasern eingearbeitet. Es wurde festgestellt, dass sich die in DE 102005048939 A1 beschriebenen Vorrichtungen so einstellen lassen, dass die erfindungsgemäß bevorzugten kurzen Verweilzeiten erzielt werden.

Das Behältnis könnte auf 300° C erwärmbar sein. Diese Ausgestaltung ermöglicht vorteilhaft eine Verwendung nahezu aller thermoplastischen faserbildenden Werkstoffe als Faserrohmaterial. Hierbei ist denkbar, dass Polyester, Polyamide, Polyurethane, Polylactide und Polyhydroxybutyrat/Polyhydroxyvalerat-Copolymere sowie native Zucker, z.B. Saccharose, oder Mischungen aus den genannten Stoffen verwendet werden. Darüber hinaus könnte das Faserrohmaterial Polyolefine oder reaktive Polymere umfassen. Denkbar ist hierbei, dass Polypropylen, mit Acrylsäure gepfropftes Polypropylen und/oder modifiziertes Polypropylen verwendet werden. Die Verwendung von biologisch abbaubaren Stoffen wie Gelatine als Faserrohmaterial ermöglicht die Erzeugung von Fasern, die problemlos entsorgbar sind. Des Weiteren können mit diesen Fasern medizinische Produkte wie Wundverbände oder Zeilwachstumsträger gefertigt werden. Alle genannten Stoffe können allein oder in Mischung mit anderen als Faserrohmaterial Verwendung finden.

Dem rotierenden Behältnis könnte eine Ablageeinrichtung zur Aufnahme von Faserrohmaterial zugeordnet sein. Die Ablageeinrichtung könnte als Plattform ausgestaltet sein, auf der die Fasern zur Bildung eines Faserflors oder Vlieses abgelegt werden können. Denkbar ist auch, dass die Ablageeinrichtung eine rotierende Einrichtung ist, auf welcher Fasern zur Beschichtung eines zylindrischen Körpers oder zur Erzeugung eines Wickelvlieses aufgenommen werden.

Zwischen der Ablageeinrichtung und dem Behältnis könnte eine elektrische Potentialdifferenz bestehen. Diese konkrete Ausgestaltung erlaubt die Fertigung von elektrostatisch aufgeladenen Fasern.

Des Weiteren ist denkbar, dass die elektrische Potentialdifferenz zur Unterstützung der Herstellung von Nanofasem verwendet wird. Hierbei addieren sich die Effekte der Zentripetalkräfte und des elektrischen Feldes, d.h. das Faserrohmaterial wird einerseits durch die Zentripetalkräfte in dünnen Fäden tangential vom rotierenden Behältnis weg geschleudert und des Weiteren durch das elektrische Feld ggf. noch weiter aufgesplittet. Insoweit ist denkbar, einen Fertigungsprozess zu realisieren, durch welchen Fasern im Nanometerbereich herstellbar sind.

Das Faserrohmaterial könnte bereits in fluidisierter Form in das Behältnis eingebracht werden. Hierdurch ist es möglich, einen kontinuierlichen Prozess durchzuführen, indem nämlich das Faserrohmaterial außerhalb des Behältnisses erwärmt wird. Denkbar ist jedoch auch, das Faserrohmaterial erst zu fluidisieren, nachdem es in das Behältnis eingebracht wurde.

Gegenstand der Erfindung ist auch ein Wundverband oder Implantat, enthaltend einen Vliesstoff nach einem der vorhergehenden Ansprüche. Die Vliesstoffe können in nach dem Stand der Technik bekannten Wundverbänden eingesetzt werden. Die Wundverbände können Trocken- oder Nassverbände sein. Bei den Trockenverbänden kann es sich beispielsweise um Materialien auf der Basis von Polypropylen handeln. Bei den nassen Verbänden sind Hydrogele und Hydrofasem bevorzugt, insbesondere aus bioresorbierbaren Materialien. Ein Hydrogel ist ein wasserenthaltendes, wasserunlösliches Polymer, dessen Moleküle chemisch, z. B. durch kovalente oder ionische Bindungen, oder physikalisch, z. B. durch Verschlaufen der Polymerketten, zu einem dreidimensionalen Netzwerk verknüpft sind. Durch hydrophile Polymerkomponenten quellen sie in Wasser unter beträchtlicher Volumenzunahme, ohne ihren stofflichen Zusammenhalt zu verlieren. i Fasermaterialien für Hydrogele sind beispielsweise Polyacrylamide, Natriumalginat, Calciumalginat, Guar Gum, Xanthan Gum, modifizierte Stärke, Pectin, Agar, Glycerylpolymethacrlylat, Polyurethangele, Carboxymethylcellulose und Hydroethylcellulose. Geeignete Fasermaterialien für Hydrofasem sind beispielsweise Natriumcarboxymethylcellulose, Calciumalginat und Calcium/Natriumalginat.

Die Wundverbände der Erfindung können auch mehrschichtig sein. Dabei können erfindungsgemäße Vliesstoffe mit weiteren, nicht erfindungsgemäßen Schichten kombiniert werden. Die Ausgestaltung hängt von der gewünschten Anwendung ab. So werden die Fasermaterialien und die biologisch aktiven Substanzen der Art der Wunde und dem Stadium der Wundheilung angepasst.

Der erfindungsgemäße Vliesstoff kann auch als Implantat oder Teil eines Implantats verwendet werden. Dabei ist die Verwendung in bioresorbierbaren oder langzeitstabilen Implantaten möglich. Beispielsweise können die Implantate bei chirurgischen Eingriffen eingesetzt werden und durch die biologischen aktiven Substanzen gezielt therapeutisch wirken.

Vliesstoffe der hier beschriebenen Art könnten im medizinischen Bereich Verwendung finden, da sie im Hinblick auf ihre Gewebestruktur und stoffliche Zusammensetzung sehr gut modifizierbar sind. Beispielsweise ist denkbar, die Gewebestruktur so einzustellen, dass sie als Wundverband fungieren können, wenn nämlich die Fasergewebestruktur mit dem menschlichen Gewebe gut verwachsen kann. Weitere medizinische Anwendungen wie die Verwendung als Zellwachstumsträger sind denkbar.

Der hier beschriebene Vliesstoff eignet sich besonders zur Herstellung eines Wattetupfers oder Tupfers, da er hinreichend stabil ist und desinfizierend wirkt.

Weitere Ausrüstungen des Vliesstoffs sind möglich. Der Vliesstoff könnte mit rekombinanten Wachstumsfaktoren, autologen Wachstumsfaktoren, insbesondere Thrombozyten-Präparationen, Adhäsionsfaktoren, insbesondere RDG-Peptiden, und/oder autologen Zellpräparationen, insbesondere Knochenmarkaspiraten ausgerüstet sein.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche, andererseits auf die nachfolgende Erläuterung bevorzugter Ausführungsbeispiele des erfindungsgemäßen Vliesstoffs anhand der Zeichnung zu verweisen.

In Verbindung mit der Erläuterung der bevorzugten Ausführungsbeispiele anhand der Zeichnung werden auch im Allgemeinen bevorzugte Ausgestaltungen und Weiterbildungen der Lehre erläutert.

### Kurzbeschreibung der Zeichnung

In der Zeichnung zeigen
- Fig. 1: eine REM-Aufnahme eines Vliesstoffs aus Gelatine, in dessen Fasern Silber als antimikrobieller Stoff in Form von nanoskaligen Partikeln verteilt ist,
- Fig. 2: eine REM-Aufnahme des Vliesstoffs aus Fig. 1 in vergrößerter Ansicht,
- Fig. 3: eine REM-Aufnahme einer Faser des Vliesstoffs aus Fig. 1 in vergrößerter Ansicht, welche einen Durchmesser von 4 µm aufweist,
- Fig. 4: eine Stereomikroskop-Aufnahme eines Vliesstoffes aus Dextran und
- Fig. 5: eine Stereomikroskop-Aufnahme des Vliesstoffes aus Fig. 4 in vergrößerter Ansicht.

### Ausführung der Erfindung

### Ausführungsbeispiel 1:

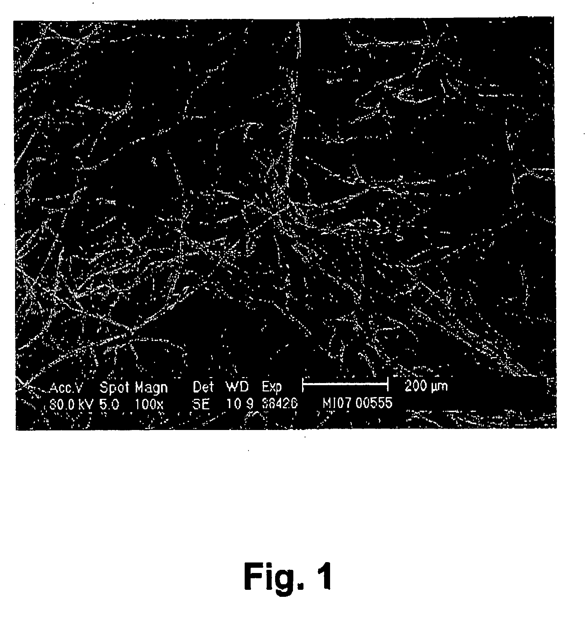

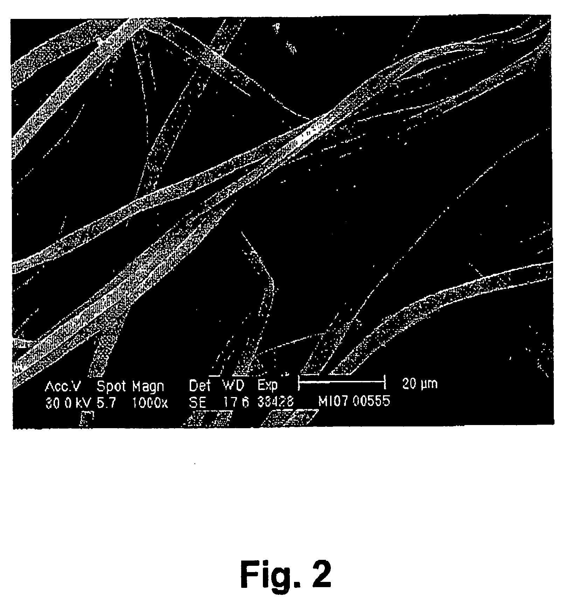

Ein Vliesstoff mit Silber als antimikrobiellem Stoff gemäß den Fig. 1 und 2 wird durch ein Rotationsspinnverfahren wie folgt hergestellt:

Zunächst wird eine 20%ige Gelatine-Lösung hergestellt. Zur Verwendung kommt eine Gelatine des Typs A PIGSKIN der Firma GELITA AG. Die Gelatine wird in Wasser eingerührt. Der Gelatine-Lösung werden 1000 ppm des Feststoffgehalts einer 5%igen wässrigen Silbersuspension zugegeben, die Silber in Form von nanoskaligen Partikeln enthält. Es wurde eine Silbersuspension der Firma RENT A SCIENTIST, des Typs AGPURE verwendet. Hierdurch entsteht eine Endkonzentration von 50 mg Silber/(kg Gelatine-Lösung).

Darauf verbleibt die Gelatine-Lösung etwa eine Stunde in Ruhe, um aufzuquellen. Anschließend wird die Gelatine-Lösung bei 60°C in einem Ultraschallbad gelöst und darauf ca. 2 Stunden auf einer Temperatur von 80-85°C gehalten. Die Partikel des Silbers können in der Gelatine-Lösung Agglomerate bilden, die durch Rühren der Gelatine-Lösung aufgelöst werden.

Die auf 80-85°C temperierte Gelatine-Lösung wird mittels einer Schlauchpumpe als Faserrohmaterial in das Behältnis einer Vorrichtung zum Rotationsspinnen gemäß der DE 102005045939 A1 geführt,

Das Behältnis hat eine Temperatur von ca. 120°C und rotiert mit einer Drehzahl von 4500 U/min, Im Behältnis befinden sich Ausnehmungen, die als Löcher mit einem Durchmesser von 0,3 mm ausgestaltet sind. Durch die Zentripetalkraft wird das Faserrohmaterial durch die Ausnehmungen gepresst und zu Fasern gesponnen, die durch eine Absaugeinrichtung verstreckt werden. Die Absaugeinrichtung befindet sich unterhalb des Behältnisses.

Nach Vernetzung der Gelatine wird ein antimikrobiell wirksamer, bioresorbierbarer Vliesstoff aus Gelatine, nämlich ein Gelatinevliesstoff, erhalten.

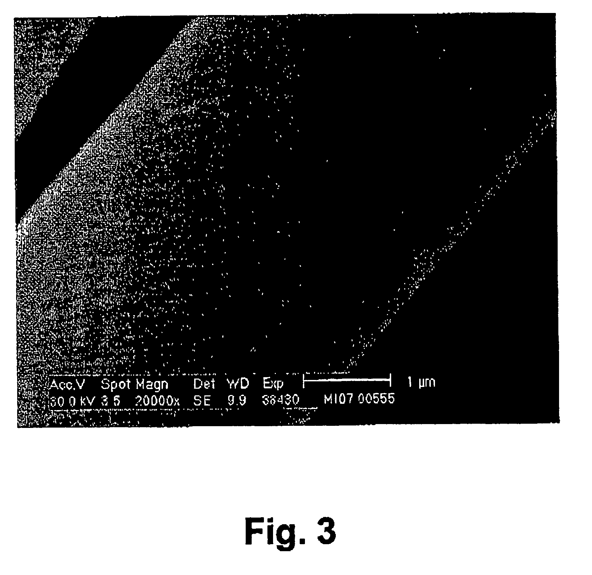

Der Vliesstoff wurde mit einem Rasterelektronenmikroskop (REM) charakterisiert. Die Fig. 1 bis 3 zeigen REM-Aufnahmen des hier beschriebenen Vliesstoffs mit unterschiedlicher Vergrößerung. Gemäß einer Charakterisierung nach ICP (gemäß EN ISO 11885) beträgt die Silberkonzentration im Vliesstoff 44 mg/kg.

### Ausführungsbeispiel 2:

Ein Vliesstoff mit Antibiotika wird durch ein Rotationsspinnverfahren wie folgt hergestellt:

Zur Herstellung eines Vliesstoffs wird zunächst eine 20%ige Gelatine-Lösung hergestellt. Zur Verwendung kommt eine Gelatine vom Typ A PIGSKIN gemäß Beispiel 1. Die Gelatine wird in Wasser eingerührt. Diese Gelatine-Lösung verbleibt zum Quellen für eine Stunde in Ruhe. Anschließend wird die Gelatine-Lösung bei 60°C in einem Ultraschallbad gelöst und dann etwa zwei Stunden auf einer Temperatur von 80-85°C gehalten.

Die auf 80-85°C temperierte Gelatine-Lösung wird mittels einer Schlauchpumpe in das Behältnis gemäß der DE 102005048939 A1 geführt. Kurz vor dem Eintritt der Gelatine-Lösung in die Ausnehmungen wird der Gelatine-Lösung eine Ampulle Gentamicin-Lösung (GENTAMICIN 40 der Firma HEXAL AG) beigemengt. Das Behältnis hat eine Temperatur von ca. 124°C und dreht mit einer Drehzahl von 4500 U/min. Durch die Zentripetalkraft wird das Faserrohmaterial aus den am Behältnis befindlichen Ausnehmungen gepresst und zu Fasern gesponnen. Die Fasern werden durch eine Absaugeinrichtung, die sich unterhalb des Behältnisses befindet, verstreckt. Nach Vernetzung der Gelatine wird ein Vliesstoff mit einem eingeschlossenen Antibiotika erhalten, welcher antimikrobiotisch wirkt und zugleich bioresorbierbar ist.

### Ausführungsbeispiel 3:

Ein Vliesstoff mit nachträglicher Antibiotika-Ausrüstung wird durch ein Rotationsspinnverfahren wie folgt hergestellt:

Zur Herstellung eines Vliesstoffs wird zunächst eine 20%ige Gelatine-Lösung hergestellt. Zur Verwendung kommt eine Gelatine vom Typ A PIGSKIN gemäß Beispiel 1. Die Gelatine wird in Wasser eingerührt. Diese Gelatine-Lösung verbleibt zum Quellen für eine Stunde in Ruhe. Anschließend wird die Gelatine-Lösung bei 60°C in einem Ultraschallbad gelöst und dann etwa zwei Stunden auf einer Temperatur von 80-85°C gehalten.

Die auf 80-85°C temperierte Gelatine-Lösung wird mittels einer Schlauchpumpe in das Behältnis gemäß der DE 102005048939.A1 geführt. Das Behältnis hat eine Temperatur von ca. 120°C und dreht mit einer Drehzahl von 4500 U/min. Durch die Zentripetalkraft wird das Faserrohmaterial aus den am Behältnis befindlichen Ausnehmungen gepresst und zu Fasern gesponnen. Die Fasern werden durch eine Absaugeinrichtung, die sich unterhalb des Behältnisses befindet, verstreckt. Nach Vemetzung der Gelatine wird der Vliesstoff mit einer Lösung von Gentamicin besprüht und danach getrocknet.

### Ausführungsbeispiel 4:

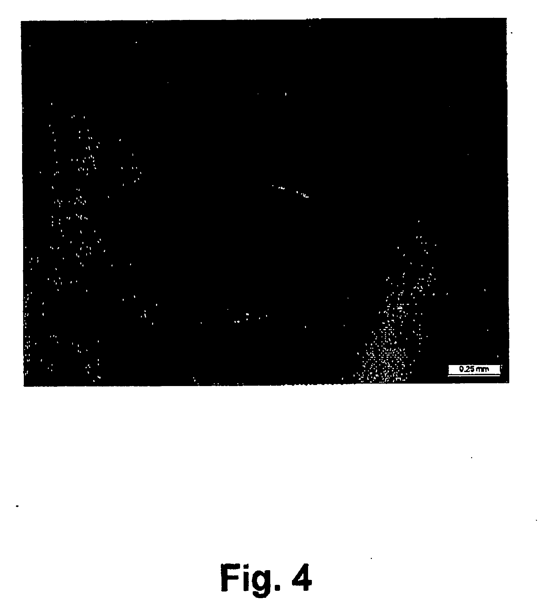

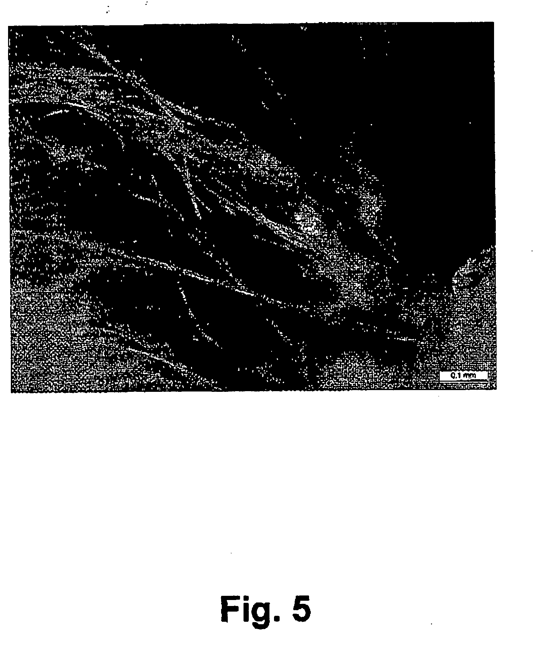

Es wird der in den Figuren 4 und 5 abgebildete erfindungsgemäße Vliesstoff aus Dextran in einem Rotationsspinnverfahren hergestellt.

Zunächst wird eine 40%ige Dextran-Lösung hergestellt, die aus 60 Teilen destilliertem Wasser und 40 Teilen Dextran (Firma PHARMACOSMOS A/S, M_{w}= 110000) besteht. Der Dextran-Lösung wird eine Silbersuspension zugegeben, die Silber in Form von nanoskaligen Partikeln enthält. Es wurde eine Silbersuspension der Firma RENT A SCIENTIST, des Typs AGPURE verwendet. Hierdurch entsteht eine Endkonzentration von 25 mg Silber /(kg Dextran-Lösung). Diese Lösung wird für etwa 10 Minuten bei 65 inkubiert und anschließend mit einer Pipette als Faserrohrmaterial in das Behältnis einer Vorrichtung zum Rotationsspinnen gemäß DE 102005048939 A1 eingeführt. Das Behältnis hat eine Temperatur von etwa 190 °C und rotiert mit einer Drehzahl von 4000 U/min. In dem Behältnis befinden sich Ausnehmungen, die als Löcher mit einem Durchmesser von 0,3 mm ausgestaltet sind. Durch die Zentripetalkraft wird das Faserohmaterial durch die Ausnehmungen gepresst und zu Fasern gesponnen, die durch die Absaugeinrichtung verstreckt werden. Die Absaugeinrichtung befindet sich unterhalb des Behältnisses. Nach der Vernetzung des Dextrans wird ein bioresorbierbarer Vliesstoff erhalten, der als Wundauflage verwendet werden kann. Ein solcher Vliesstoff ist in den Fig. 4 und 5 gezeigt.

Bei den Ausführungsbeispielen 1 bis 4 betrug die Verweildauer des Faserrohmaterials im Behältnis maximal 5 Sekunden. Weitere Untersuchungen haben ergeben, dass in größeren Behältnissen eine maximale Verweildauer von 9,3 Sekunden sinnvoll ist.

Hinsichtlich weiterer vorteilhafter Ausgestaltungen und Weiterbildungen der erfindungsgemäßen Lehre wird einerseits auf den allgemeinen Teil der Beschreibung und andererseits auf die beigefügten Patentansprüche verwiesen.

Abschließend sei ganz besonders hervorgehoben, dass die zuvor rein willkürlich ausgewählten Ausführungsbeispiele lediglich zur Erörterung der erfindungsgemäßen Lehre dienen, diese jedoch nicht auf diese Ausführungsbeispiele einschränken.

## Patentansprüche

1. Rotationsspinnverfahren zur Herstellung eines Vliesstoffs umfassend Fasern aus einem Faserrohmaterial, welches natürliche Polymere aufweist, wobei die Fasern mit einer biologisch aktiven Substanz ausgerüstet sind, die in den Fasern verteilt ist, wobei Faserrohmaterial in ein Behältnis gegeben wird, wobei das Behältnis in Rotation versetzt wird und wobei das fluidisierte Faserrohmaterial durch Zentripetalkräfte aus dem Behältnis in Form von Fasern ausgetragen wird, **dadurch gekennzeichnet, dass** die durchschnittliche Verweilzeit des Faserrohmaterials in dem Behältnis kleiner als 10 Sekunden, insbesondere kleiner als 1 Sekunde ist.

2. Rotationsspinnverfahren nach Anspruch 1, **dadurch gekennzeichnet, dass** die austretenden Fasern gerichtet, kontaktlos geführt werden.

3. Rotationsspinnverfahren nach einem der Ansprüche 1 bis 2, **dadurch gekennzeichnet, dass** die austretenden Fasern durch eine Absaugeinrichtung geführt werden.

4. Rotationsspinnverfahren nach einem der Ansprüche 1 bis 3, **dadurch gekennzeichnet, dass** Fasern hergestellt werden, die einen Durchmesser von 0,3 bis 500 µm aufweisen.

5. Rotationsspinnverfahren nach einem der Ansprüche 1 bis 4, **dadurch gekennzeichnet, dass** die Austrittsbereiche des Behältnisses als Durchgänge mit einem Durchmesser bis 500 µm ausgestaltet sind.

6. Rotationsspinnverfahren nach einem der Ansprüche 1 bis 5, **dadurch gekennzeichnet, dass** die Fasern auf einer Ablageeinrichtung abgelegt werden, wobei zwischen der Ablageeinrichtung und dem Behältnis eine elektrische Potentialdifferenz besteht.

7. Vliesstoff, umfassend Fasern aus einem Faserrohmaterial, wobei die Fasern mit einer biologisch aktiven Substanz ausgerüstet sind, wobei die Fasern mit einem Rotationsspinnverfahren nach einem oder mehreren der Ansprüche 1 bis 6 hergestellt und die biologisch aktive Substanz in den Fasern verteilt ist.

8. Vliesstoff nach einem der vorhergehenden Ansprüche, **dadurch gekennzeichnet, dass** das Faserrohmaterial und/oder die biologisch aktive Substanz oberhalb 150 °C derart instabil ist, dass das Material sich innerhalb von 1 Minute, insbesondere 10 Sekunden, bei dieser Temperatur so verändert, dass die Verwendbarkeit als Fasermaterial zur Erzeugung eines Vliesstoffes oder die biologische Aktivität um mindestens 10% abnimmt.

9. Vliesstoff nach einem der vorhergehenden Ansprüche, **dadurch gekennzeichnet, dass** das Faserrohmaterial ausgewählt ist aus der Gruppe bestehend aus natürlichen Polymeren, synthetischen Polymeren und Polymeren aus fossilen Rohmaterialien, jeweils in unmodifzierter und/oder modifizierter Form.

10. Vliesstoff nach Anspruch 9, **dadurch gekennzeichnet, dass** die natürlichen Polymere Polypeptide, Polysaccharide, Polyhydroxyester und Polynucleotide sind.

11. Vliesstoff nach Anspruch 10, **dadurch gekennzeichnet, dass** das Polypeptid Gelatine, Collagen, Fibrin oder Casein ist.

12. Vliesstoff nach Anspruch 10, **dadurch gekennzeichnet, dass** das Polysaccharid Dextran, Chitosan, Chitin, Cellulose, Stärke, ein Hyaluronsäurederivat oder ein Alginat ist.

13. Vliesstoff nach einem der vorhergehenden Ansprüche, **dadurch gekennzeichnet, dass** die biologisch aktive Substanz ein Antiseptikum, ein antimikrobiell wirksamer Stoff, ein Antibiotikum, ein Medikament, ein Wachstumsfaktor, ein Enzym und/oder eine Zelle ist.

14. Vliesstoff nach einem der vorhergehenden Ansprüche, **dadurch gekennzeichnet, dass** der Vliesstoff bioresorbierbar ist.

15. Vliesstoff nach einem der vorhergehenden Anspruch, **dadurch gekennzeichnet, dass** einige Fasern miteinander verdrillt sind oder eine verdrillte Struktur aufweisen.

16. Vliesstoff nach einem der vorhergehenden Ansprüche, **dadurch gekennzeichnet, dass** die Fasern ausschließlich aus Gelatine oder Dextran oder Derivaten der Gelatine oder des Dextrans gefertigt sind, wobei in und/oder auf den Fasern ein antimikrobieller Stoff und/ oder ein Antibiotikum vorliegt.

17. Vliesstoff nach einem der vorhergehenden Ansprüche, **dadurch gekennzeichnet, dass** die biologisch aktive Substanz in den Fasern nanoskalig vorliegt.

18. Vliesstoff nach einem der vorhergehenden Ansprüche, **dadurch gekennzeichnet, dass** die biologisch aktive Substanz auf den Fasen verteilt ist.

19. Vliesstoff nach einem der vorhergehenden Ansprüche, **dadurch gekennzeichnet, dass** die biologisch aktive Substanz Silber enthält.

20. Vliesstoff nach einem der vorhergehenden Ansprüche, **dadurch gekennzeichnet, dass** zumindest ein Teil der Fasern als Nanofasern ausgestaltet ist.

21. Vliesstoff nach einem der vorhergehenden Ansprüche, **gekennzeichnet durch** eine Reißfestigkeit bei einem spezifischen Flächengewicht von 140 bis 180 g/m² im trockenen Zustand von 0,10 N/mm² oder mehr und eine Reißdehnung in hydratisiertem Zustand von 100%, bevorzugt von 200% oder mehr.

22. Vliesstoff nach einem der vorhergehenden Ansprüche, **gekennzeichnet durch** eine offene Porenstruktur mit einer Luftdurchlässigkeit von 0,5 l/min*cm², gemäß DIN 9237

23. Wundverband, Wattetupfer oder Implantat, enthaltend einen Vliesstoff nach einem der vorhergehenden Ansprüche.

24. Verwendung eines Vliesstoffs nach einem der Ansprüche 7 bis 22 zur Herstellung eines Arzneimittels für die Wundheilung.

25. Verwendung eines Vliesstoffs nach einem der Ansprüche 7 bis 22 zur Herstellung eines Wattetupfers, Wundverbands oder Implantats

## Claims

1. Rotational spinning process for producing a fibrous nonwoven web fabric comprising fibres formed from a fibre raw material comprising natural polymers, wherein the fibres have been endowed with a biologically active substance which has been distributed in the fibres, wherein fibre raw material is introduced into a receptacle, wherein the receptacle is made to rotate and wherein centripetal forces carry the fluidized fibre raw material out of the receptacle in the form of fibres, **characterized in that** the average residence time of the fibre raw material in the receptacle is less than 10 seconds, in particular less than 1 second.

2. Rotational spinning process according to Claim 1, **characterized in that** the exiting fibres are guided in a directed and contactless manner.

3. Rotational spinning process according to either of Claims 1 and 2, **characterized in that** the exiting fibres are guided by a sucking device.

4. Rotational spinning process according to any of Claims 1 to 3, **characterized in that** fibres produced have a diameter in the range from 0.3 to 500 µm.

5. Rotational spinning process according to any of Claims 1 to 4, **characterized in that** the exit regions of the receptacle are configured as apertures up to 500 µm in diameter.

6. Rotational spinning process according to any of Claims 1 to 5, **characterized in that** the fibres are laid down on a laydown device, wherein there is a difference in electric potential between the laydown device and the receptacle.

7. Fibrous nonwoven web fabric comprising fibres formed from a fibre raw material, wherein the fibres have been endowed with a biologically active substance, wherein the fibres have been produced using a rotational spinning process according to one of more of Claims 1 to 6 and the biologically active substance has been dispersed in the fibres.

8. Fibrous nonwoven web fabric according to any preceding claim, **characterized in that** the fibre raw material and/or the biologically active substance are so unstable at above 150°C that the material changes within 1 minute, in particular 10 seconds, at this temperature such that the utility as a fibre material for producing a fibrous nonwoven web fabric or the biological activity decreases by not less than 10%.

9. Fibrous nonwoven web fabric according to any preceding claim, **characterized in that** the fibre raw material is selected from the group consisting of natural polymers, synthetic polymers and polymers derived from fossil raw materials, each in modified and/or unmodified form.

10. Fibrous nonwoven web fabric according to Claim 9, **characterized in that** the natural polymers are polypeptides, polysaccharides, polyhydroxyesters and polynucleotides.

11. Fibrous nonwoven web fabric according to Claim 10, **characterized in that** the polypeptide is gelatin, collagen, fibrin or casein.

12. Fibrous nonwoven web fabric according to Claim 10, **characterized in that** the polysaccharide is dextran, chitosan, chitin, cellulose, starch, a hyaluronic acid derivative or an alginate.

13. Fibrous nonwoven web fabric according to any preceding claim, **characterized in that** the biologically active substance is an antiseptic, an antimicrobial, an antibiotic, a medicament, a growth factor, an enzyme and/or a cell.

14. Fibrous nonwoven web fabric according to any preceding claim, **characterized in that** the fibrous nonwoven web fabric is bioresorbable.

15. Fibrous nonwoven web fabric according to any preceding claim, **characterized in that** some fibres are twisted together or have a twisted structure.

16. Fibrous nonwoven web fabric according to any preceding claim, **characterized in that** the fibres are made exclusively of gelatin or dextran or derivatives of gelatin or of dextran, wherein an antimicrobial and/or an antibiotic is present in and/or on the fibres.

17. Fibrous nonwoven web fabric according to any preceding claim, **characterized in that** the biologically active substance is present in the fibres in nanoscale form.

18. Fibrous nonwoven web fabric according to any preceding claim, **characterized in that** the biologically active substance is distributed on the fibres.

19. Fibrous nonwoven web fabric according to any preceding claim, **characterized in that** the biologically active substance contains silver.

20. Fibrous nonwoven web fabric according to any preceding claim, **characterized in that** at least some of the fibres are configured as nanofibres.

21. Fibrous nonwoven web fabric according to any preceding claim, **characterized by** a breaking strength of 0.10 N/mm² or more in the dry state at a specific basis weight of 140 to 180 g/m² and a breaking extension of 100% and preferably of 200% or more in the hydrated state.

22. Fibrous nonwoven web fabric according to any preceding claim, **characterized by** an open porous structure having a DIN 9237 air permeability of 0.5 l/min*cm².

23. Wound dressing, swab or implant containing a fibrous nonwoven web fabric according to any preceding claim.

24. Use of a fibrous nonwoven web fabric according to any of Claims 7 to 22 in the manufacture of a medicinal product for wound healing.

25. Use of a fibrous nonwoven web fabric according to any of Claims 7 to 22 in the manufacture of a swab, wound dressing or implant.

## Revendications

1. Procédé de filage par rotation pour la fabrication d'un non-tissé comprenant des fibres en un matériau brut fibreux, qui comprend des polymères naturels, les fibres étant munies d'une substance biologiquement active qui est répartie dans les fibres, le matériau brut fibreux étant introduit dans un contenant, le contenant étant mis en rotation et le matériau brut fibreux fluidisé étant déchargé du contenant sous la forme de fibres par des forces centripètes, **caractérisé en ce que** le temps de séjour moyen du matériau brut fibreux dans le contenant est inférieur à 10 secondes, notamment inférieur à 1 seconde.

2. Procédé de filage par rotation selon la revendication 1, **caractérisé en ce que** les fibres sortantes sont conduites d'une manière dirigée, sans contact.

3. Procédé de filage par rotation selon l'une quelconque des revendications 1 à 2, **caractérisé en ce que** les fibres sortantes traversent un dispositif d'aspiration.

4. Procédé de filage par rotation selon l'une quelconque des revendications 1 à 3, **caractérisé en ce que** des fibres présentant un diamètre de 0,3 à 500 µm sont fabriquées.

5. Procédé de filage par rotation selon l'une quelconque des revendications 1 à 4, **caractérisé en ce que** les zones de sortie du contenant sont configurées sous la forme de passages d'un diamètre de jusqu'à 500 µm.

6. Procédé de filage par rotation selon l'une quelconque des revendications 1 à 5, **caractérisé en ce que** les fibres sont déposées sur un dispositif de dépôt, une différence de potentiel électrique existant entre le dispositif de dépôt et le contenant.

7. Non-tissé, comprenant des fibres en un matériau brut fibreux, les fibres étant munies d'une substance biologiquement active, les fibres étant fabriquées par un procédé de filage par rotation selon une ou plusieurs des revendications 1 à 6 et la substance biologiquement active étant répartie dans les fibres.

8. Non-tissé selon l'une quelconque des revendications précédentes, **caractérisé en ce que** le matériau brut fibreux et/ou la substance biologiquement active sont instables au-dessus de 150 °C de sorte que le matériau se modifie à cette température en l'espace de 1 minute, notamment de 10 secondes, de manière à ce que l'applicabilité en tant que matériau fibreux pour la formation d'un non-tissé ou l'activité biologique diminue d'au moins 10 %.

9. Non-tissé selon l'une quelconque des revendications précédentes, **caractérisé en ce que** le matériau brut fibreux est choisi dans le groupe constitué par les polymères naturels, les polymères synthétiques et les polymères de matériaux bruts fossiles, à chaque fois sous forme non modifiée et/ou modifiée.

10. Non-tissé selon la revendication 9, **caractérisé en ce que** les polymères naturels sont des polypeptides, des polysaccharides, des polyhydroxyesters et des polynucléotides.

11. Non-tissé selon la revendication 10, **caractérisé en ce que** le polypeptide est la gélatine, le collagène, la fibrine ou la caséine.

12. Non-tissé selon la revendication 10, **caractérisé en ce que** le polysaccharide est le dextrane, le chitosan, la chitine, la cellulose, l'amidon, un dérivé d'acide hyaluronique ou un alginate.

13. Non-tissé selon l'une quelconque des revendications précédentes, **caractérisé en ce que** la substance bioloqiquement active est un antiseptique, une substance à action antimicrobienne, un antibiotique, un médicament, un facteur de croissance, une enzyme et/ou une cellule.

14. Non-tissé selon l'une quelconque des revendications précédentes, **caractérisé en ce que** le non-tissé est biorésorbable.

15. Non-tissé selon l'une quelconque des revendications précédentes, **caractérisé en ce que** plusieurs fibres sont torsadées les unes avec les autres ou présentent une structure torsadée.

16. Non-tissé selon l'une quelconque des revendications précédentes, **caractérisé en ce que** les fibres sont exclusivement en gélatine ou dextrane ou dérivés de gélatine ou de dextrane, une substance antimicrobienne et/ou un antibiotique étant présent dans et/ou sur les fibres.

17. Non-tissé selon l'une quelconque des revendications précédentes, **caractérisé en ce que** la substance biologiquement active est présente à l'échelle nanométrique dans les fibres.

18. Non-tissé selon l'une quelconque des revendications précédentes, **caractérisé en ce que** la substance biologiquement active est répartie sur les fibres.

19. Non-tissé selon l'une quelconque des revendications précédentes, **caractérisé en ce que** la substance biologiquement active contient de l'argent.

20. Non-tissé selon l'une quelconque des revendications précédentes, **caractérisé en ce qu'**au moins une partie des fibres sont configurées sous la forme de nanofibres.

21. Non-tissé selon l'une quelconque des revendications précédentes, **caractérisé par** une résistance à la rupture à un poids superficiel spécifique de 140 à 180 g/m² à l'état sec de 0,10 N/mm² ou plus et un allongement à la rupture à un état hydraté de 100 %, de préférence de 200 % ou plus.

22. Non-tissé selon l'une quelconque des revendications précédentes, **caractérisé par** une structure de pores ouverte d'une perméabilité à l'air de 0,5 1/min*cm² selon DIN 9237.

23. Pansement, tampon d'ouate ou implant, contenant un non-tissé selon l'une quelconque des revendications précédentes.

24. Utilisation d'un non-tissé selon l'une quelconque des revendications 7 à 22 pour la fabrication d'un médicament pour la cicatrisation.

25. Utilisation d'un non-tissé selon l'une quelconque des revendications 7 à 22 pour la fabrication d'un tampon d'ouate, d'un pansement ou d'un implant.
